# FASCICULE DE BREVET EUROPEEN

(11) **EP 2 943 785 B1**
(45) Date de publication et mention de la délivrance du brevet: **25.10.2017**
(21) Numéro de dépôt: 14700566.4
(22) Date de dépôt: 06.01.2014
(51) Int. Cl.: G01N 27/403, G01N 30/72, G01N 30/88

(54) **DISPOSITIF ET PROCÉDÉ DE SYNTHÈSE D'ESPÈCES INTERMÉDIAIRES D'UNE ENTITÉ CHIMIQUE PAR VOIE ÉLECTROCHIMIQUE**
VORRICHTUNG UND VERFAHREN ZUR ELEKTROCHEMISCHEN SYNTHESE VON ZWISCHENSPEZIES EINER CHEMISCHEN EINHEIT
DEVICE AND METHOD FOR ELECTROCHEMICALLY SYNTHESIZING INTERMEDIATE SPECIES OF A CHEMICAL ENTITY

(30) Priorité: 08.01.2013 FR 1350154
(43) Date de publication de la demande: 18.11.2015
(73) Titulaire: Centre National de la Recherche Scientifique (C.N.R.S.), 75016 Paris (FR); Universite de Nantes, 44000 Nantes (FR)
(72) Inventeur: BOUJTITA, Mohammed, F-44300 Nantes (FR); BUSSY, Ugo, 85520 Saint Vincent Sur Jard (FR)
(74) Mandataire: Lavoix
(86) Numéro de dépôt international: PCT/EP2014/050098
(87) Numéro de publication internationale: WO 2014/108372

(56) Documents cités:
- WO-A1-97/41429
- WO-A2-03/081208
- US-A- 4 133 726
- US-A- 4 511 659
- LOHMANN W ET AL: "On-line electrochemistry/liquid chromatography/mass spectrometry for the simulation of pesticide metabolism", JOURNAL OF THE AMERICAN SOCIETY FOR MASS SPECTROMETRY, vol. 20, no. 1, janvier 2009 (2009-01), pages 138-145, XP026674137, ISSN: 1044-0305, DOI: 10.1016/J.JASMS.2008.09.003 [extrait le 2008-09-07]
- BAUMANN A ET AL: "Online electrochemistry/mass spectrometry in drug metabolism studies: principles and applications", EXPERT OPINION ON DRUG METABOLISM & TOXICOLOGY, vol. 6, no. 6, juin 2010 (2010-06), pages 715-731, XP009173066, ISSN: 1744-7607

## Description

La présente invention concerne un dispositif et un procédé de synthèse par voie électrochimique d'espèces intermédiaires, telles que des métabolites.

L'invention trouve une application dans le domaine de l'identification structurale des produits d'oxydation, par exemple par spectrométrie de masse, RMN, Infra-Rouge. Notamment, dans le domaine pharmaceutique, l'invention s'applique à la réalisation des tests pharmacologiques et toxicologiques, plus précisément pour l'étude du devenir oxydatif de certains médicaments en vue d'évaluer la stabilité, l'activité chimique et la réactivité biologique des principales espèces intermédiaires. Egalement, dans le domaine de l'agroalimentaire, l'invention s'applique à l'élucidation de la dégradation oxydative ou photo-oxydative d'additifs alimentaires tels que les conservateurs, colorants, antioxydants. Dans encore un autre domaine, celui de l'environnement, l'invention s'applique à la prédiction du devenir des polluants émergeants comme les médicaments, détergents, dérivés phénoliques.

La prédiction des risques posés par de nombreuses entités chimiques (tels que les xénobiotiques) pour la santé humaine et son environnement est considérée aujourd'hui comme un enjeu sociétal majeur. De nombreux xénobiotiques comme les médicaments, polluants émergeants, pesticides, conservateurs, additifs alimentaires et autres, ont montré qu'ils pouvaient provoquer des effets secondaires majeurs, comme en témoignent des exemples de retraits de certains médicaments, conservateurs alimentaires et produits phytosanitaires du marché.

Dans ce contexte, le développement de nouveaux outils analytiques in vitro mimant le métabolisme oxydatif constitue actuellement un axe émergent d'investigation incontournable pour la prédiction d'éventuels effets toxiques de certaines entités chimiques. Ces nouveaux outils se basent essentiellement sur la prédiction de schémas de dégradation oxydative qu'est susceptible de subir un xénobiotique (Donato, M. T. ; Castell, J. V. ; Gomez-Lechon, M. J., Characterization of drug metabolizing activities in pig hepatocytes for use in bioartificial liver devices: comparison with other hepatic cellular models. Journal of Hepatology 1999, 31, (3), 542-549 ; Dong H., Haining, R. L., Thummel, K. E.; Rettie, A. E.; Nelson, S. D.; Involvement of human cytochrome p450 2D6 in the bioactivation of acetaminophen. Drug Metab Dispos 2000, 28, (12), 1397-400 ; Ferchaud, V; Le, B. B., Montrade, M-P.; Maume, D. ; Monteau, F.; André, F., Gas chromatographic-mass spectrometric identifcation of main metabolites of stanozolol in cattle after oral and subcutaneous administration. J. Chromatogr., B Biomed. Sci Appl. 1997, 695, (2) 269-277).

Plusieurs modèles biologiques utilisés in vitro ont été explorés pour l'étude de la métabolisation oxydative des xénobiotiques (Henderson, M.C, Siddens, L.K, Morré, J.T, Krueger, S.K, Williams, D.E. Metabolism of the anti-tuberculosis drug ethionamide by mouse and human FMO1, FMO2 and FMO3 and mouse and human lung microsomes. Toxicology and Applied Pharmacology 2008, 233, (3), 420-427 ; Yun, C-H. Miller, G.P, Guengerich, F.P. Rate-Determining Steps in Phenacetin Oxidations by Human Cytochrome P450 1A2 and Selected Mutants. Biochemistry 200, 39, (37), 11319-11329).

Nous pouvons citer par exemple l'utilisation des coupes de foie pour l'étude de certains métabolismes en vue de l'identification des différents métabolites. L'utilisation d'hépatocytes, aujourd'hui commercialement disponibles, connaît également un grand succès dans ce domaine.

De plus, grâce au développement de la biologie moléculaire et la mise sur le marché de nombreuses enzymes recombinées, une préférence plus significative est aujourd'hui relatée pour l'utilisation d'enzymes de la famille des cytochrome-P450 (Dong H., Haining, R. L., Thummel, K. E.; Rettie, A. E.; Nelson, S. D.; involvement of human cytochrome p450 2D6 in the bioactivation of acetaminophen. Drug Metab Dispos 2000, 28, (12), 1397-400 ; Anzenbacher, P. Anzenbacherova, E. Cytochromes P450 and metabolism of xenobiotics. Celle. Mol. Life Sci. 2001, 58, (5/6), 737-747 ; Delaforge, M. Pruvost, A. Perrin, L. Andre, F. Cytochrome P450-mediated oxidation of glucuronide derivatives: exampleof estradiol-17Î2-glucuronide oxidation to 2-hydroxy-estrdiol-17Î2-glucuronide by CYP 2C8 Drug Metab Dispos 2005, 33, (3), 466-473 ; Isin, E. M. Guengerich, F.P., Complex reactions catalyzed by cytochrome P450 enzymes biochimica et Biophysica Acta (BBA)- General Subjects 2007, 1770, (3), 314-329).

Ces modèles biologiques sont considérés comme des outils de choix pour l'étude du métabolisme oxydatif ; ils permettent non seulement d'apporter une compréhension nouvelle des voies oxydatives, mais également d'élucider le mode d'action ou d'expliquer les raisons d'une éventuelle toxicité d'une entité chimique. D'usage très répandu dans l'industrie pharmaceutique et dans de nombreux laboratoires de recherche, ces tests in vitro constituent des modèles simplifiés en comparaison aux essais in vivo, et permettent de mettre en place les bases de modèles expérimentaux in vivo, notamment dans le cas de développement de médicaments candidats, et dans le cas de l'étude des effets de polluants émergeants sur la santé humaine et l'environnement.

Il est à noter également que le développement des essais in vitro dans le domaine de la dégradation oxydative durant cette dernière décennie doit également son succès à l'évolution de l'instrumentation analytique au sein même de ces essais, avec l'utilisation de techniques tels que les techniques d'extractions (SPE), les colonnes de plus en plus performantes, le couplage HPLC et la spectrométrie de masse.

Cependant, leur avantage n'anéantit pas certaines contraintes inhérentes aux techniques utilisées in vitro : lenteur d'analyse, difficulté de caractérisation structurale des espèces intermédiaires issues de la dégradation oxydative d'un xénobiotique (faibles quantités générées), faible compatibilité de solvants organiques (solubilisation des xénobiotiques) avec l'utilisation de matériels biologiques (cellules, enzymes et autres)...

Certains de ces problèmes ont été contournés en utilisant des procédés chimiques (Chorghade, M. S.; Hill, D. R. ; Lee, E. C.; Pariza, R. J.; Dolphin, D. H.; Hino, F.; Zhang, L.-Y., Metalloporphyrins as chemical mimics of cytochrome P-450 systems. Pure Appl. Chem. 1996, 68, (3), 753-756) et électrochimiques (Karst, U. ; Diehl, G.; Hayen, H. Coupling electrochemistry to mass spectrometry and high performance liquid chromatography.2003 ; Karst, U., Analytical methods: Electrochemistry/mass spectrometry (EC/MS) - a new tool to study drug metabolism and reaction mechanisms. Angew. Chem., Int. Ed. 2004, 43, (19), 2476-2478).

En effet, il a été montré qu'une cellule électrochimique (CE) classique à trois électrodes associées aux performances de la chromatographie en phase liquide (CL) et de la spectrométrie de masse (couplage CE-CL-SM) pouvait mimer certaines réactions du métabolisme oxydatif, notamment celles initiées et catalysées par la familles de cytochrome-P450, comme par exemple la N-désalkylation, la O-désalkylation, époxidation, l'oxydation des thiols, des alcools, la déshydrogénation des cycles aromatiques (Nouri-Nigjeh, E. Permentier, H. P. Bischoff, R. Bruins, A. P., Electrochemical Oxidation by Square-Wave Potential Pulses in the Imitation of Oxidative Drug Metabolism. Anal. Chem. 2011, (83), 14, 5519. Nouri-Nigjeh, E. Bruins, A. P. Bischoff, R. Permentier, H. P., Electrocatalytic oxidation of hydrogen peroxide on a platinum electrode in the imitation of oxidative drug metabolism of lidocaïne. Analyst. 2012, (137), 4698.).

Cependant, de nombreux points restent encore à explorer. Par exemple, nous constatons une absence de dispositif de synthèse en quantité suffisante et sous un état stable des principales espèces intermédiaires issues de la dégradation oxydative d'un xénobiotique. Ceci limite, d'une part, l'utilisation de la RMN pour l'élucidation avec plus d'exactitude la structure chimique des différentes espèces, et d'autre part, la mise en oeuvre des essais de prédétermination de la concentration seuil à l'évaluation du potentiel inhibiteur ou toxique des principales espèces issues de l'oxydation d'un xénobiotique.

Un des objets de l'invention est donc d'apporter une solution aux problèmes et inconvénients précités.

L'invention se rapporte ainsi, à un dispositif de synthèse d'espèces intermédiaires d'une entité chimique par voie électrochimique, selon la revendication 1. Le document JOURNAL OF THE AMERICAN SOCIETY FOR MASS SPECTROMETRY, vol. 20, no. 1, janvier 2009, pages 138-145, XP026674137, ISSN 1044-0305; LOHMANN W et al: "On-line electrochemistry/liquid chromatography/mass spectrometry for the simulation of pesticide metabolism" décrit un tel dispositif avec toutes les caractéristiques du préambule de la revendication principale.

Le dispositif comprend une cellule électrochimique d'oxydation qui comporte au moins une première électrode de travail. Cette cellule électrochimique d'oxydation est apte, lorsque la première électrode de travail est soumise à un potentiel électrique, à générer les espèces intermédiaires par oxydation d'une solution introduite dans la cellule électrochimique d'oxydation et comprenant l'entité chimique en question.

Le dispositif comprend également une cellule électrochimique de stabilisation comportant au moins une deuxième électrode de travail, distincte de la première électrode de travail. Cette cellule électrochimique de stabilisation est apte, lorsque la deuxième électrode de travail est soumise à un potentiel électrique, à réaliser une réduction d'une solution.

Cette cellule électrochimique de stabilisation est reliée en série à la cellule électrochimique d'oxydation en sorte de permettre la réduction continue des espèces intermédiaires générées dans la cellule électrochimique d'oxydation.

La cellule électrochimique d'oxydation comprend une contre-électrode disposée parallèlement à l'électrode de travail correspondante et maintenue espacée de cette dernière au moyen d'un élément d'espacement, tel qu'un joint de silicone. Dans des variantes de réalisation le dispositif comprend les caractéristiques présentées ci-après. De préférence, l'épaisseur de l'élément d'espacement est comprise entre 0.4 et 1.1 mm.

Dans le cas où au moins la cellule électrochimique de stabilisation comprend une contre-électrode, la face de cette contre-électrode en vis-à-vis de la deuxième électrode de travail est recouverte d'un film poreux.

Les électrodes de travail et/ou la ou les contre-électrodes sont de forme sensiblement rectangulaire.

La cellule électrochimique d'oxydation et/ou la cellule électrochimique de stabilisation comprennent une électrode de pseudo-référence.

De préférence, la ou les électrodes de pseudo-référence sont disposées sur l'une des faces des électrodes de travail respectives, de préférence sur tout ou partie du pourtour de l'une des faces de ces électrodes de travail, et isolées électriquement de ces dernières par des couches isolantes respectives.

Ces couches isolantes sont par exemple être déposées par sérigraphie.

Le dispositif comprend un corps. Ce corps comprend un premier et un deuxième logements destinés à recevoir les cellules électrochimiques respectivement d'oxydation et de stabilisation.

Ces premier et deuxième logements sont formés par deux espaces disposés de part et d'autre d'un élément central en forme de « H ».

Le corps est fermé par une plaque supérieure du côté des contre-électrodes, et par une plaque inférieure du côté des électrodes de travail.

La plaque supérieure est pourvue de deux orifices en sa partie qui recouvre la cellule électrochimique d'oxydation. L'un de ces orifices constitue un orifice d'entrée dans la cellule électrochimique d'oxydation. L'autre constitue un orifice de sortie de la cellule électrochimique d'oxydation.

Cette plaque supérieure est également pourvue de deux autres orifices en sa partie qui recouvre la cellule électrochimique de stabilisation. L'un de ces orifices constitue un orifice d'entrée dans la cellule électrochimique de stabilisation. L'autre constitue un orifice de sortie de la cellule électrochimique de stabilisation.

L'orifice de sortie de la cellule électrochimique d'oxydation est relié à l'orifice d'entrée dans la cellule électrochimique de stabilisation.

La plaque inférieure est pourvue de deux éléments de fixation aptes à permettre la fixation respectivement des première et deuxième électrodes de travail.

Ainsi, le dispositif de l'invention permet la synthèse des espèces intermédiaires en quantité suffisante, notamment pour la réalisation d'analyses structurales ou encore de tests pharmacologiques et/ou toxicologiques.

L'invention se rapporte également, à un procédé de synthèse d'espèces intermédiaires d'une entité chimique par voie électrochimique selon la revendication 11. Le procédé comprend une étape de génération des espèces intermédiaires par oxydation d'une solution contenant l'entité chimique dans une cellule électrochimique d'oxydation.

Le procédé comprend également une étape de stabilisation des espèces intermédiaires générées par réduction dans une cellule électrochimique de stabilisation distincte de, et reliée en série à, la cellule électrochimique d'oxydation.

Ainsi, le procédé est simple, demande peu de manipulations et permet de synthétiser et stabiliser en quantités suffisantes les principales entités intermédiaires issues de l'oxydation d'une molécule d'intérêt.

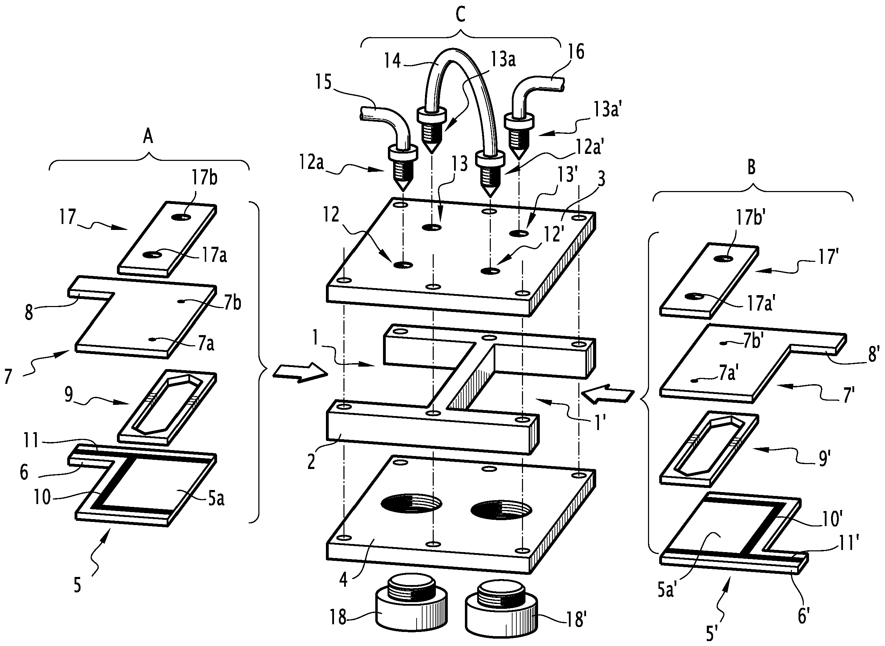

D'autres caractéristiques et avantages de l'invention apparaîtront plus clairement à la lecture de la description ci-après d'une variante préférée de réalisation de l'invention, laquelle est donnée à titre d'exemple non limitatif et en référence au dessin annexé dont la figure unique est une vue éclatée d'un dispositif selon l'invention.

Le dispositif comprend deux cellules électrochimiques (A) et (B) qui viennent se loger respectivement dans les logements 1 et 1' d'un corps (C).

Dans l'exemple représenté sur la figure, les deux cellules électrochimiques (A) et (B) se logent de part et d'autre d'un élément central 2 en forme de « H » qui délimite les deux logements 1 et 1'.

Le corps (C) est fermé par une plaque supérieure 3 et une plaque inférieure 4. L'élément central 2 est enserré entre la plaque supérieure 3 et la plaque inférieure 4. L'ensemble plaque supérieure 3, élément central 2 et plaque inférieure 4 peut être maintenu solidaire par un ou plusieurs éléments de fixation tels que des vis non représentées sur la figure par souci de clarté (seuls les axes et les trous traversant ont été représentés dans la plaque supérieure 3, l'élément central 2 et la plaque inférieure 4).

Alternativement, deux plaques supérieures distinctes pourraient être utilisées pour fermer le corps (C) respectivement au niveau de l'une et l'autre des deux cellules électrochimiques (A) et (B). De même, deux plaques inférieures distinctes pourraient être utilisées pour fermer le corps (C) respectivement au niveau de l'une et l'autre des deux cellules électrochimiques (A) et (B)

La première cellule électrochimique (A) permet, lorsqu'elle est soumise à un potentiel électrique, la génération d'espèces intermédiaires d'une entité chimique par oxydation d'une solution introduite à l'intérieur et qui contient cette entité chimique.

Cette cellule électrochimique d'oxydation (A) comprend une électrode de travail 5 qui est de préférence de forme sensiblement rectangulaire, avec une extension 6 constituant un connecteur 6 permettant de connecter l'électrode de travail 5 à un potentiostat.

Une contre-électrode 7, également de préférence de forme sensiblement rectangulaire, est disposée en vis-à-vis de l'électrode de travail 5, parallèlement à cette dernière. Cette contre-électrode 7 est pourvue d'une extension 8 constituant un connecteur 8 permettant de la connecter au potentiostat.

L'électrode de travail 5 et la contre-électrode 7 sont maintenues espacées l'une de l'autre par un élément d'espacement 9, qui peut par exemple être de type joint de silicone.

On choisira un espacement de préférence compris entre 0,4 et 1,1 mm, voire entre 0,5 et 1 mm.

Ainsi, l'espace maintenu entre l'électrode de travail 5 et la contre-électrode 7 par l'intermédiaire de l'élément d'espacement 9 forme un volume en contact à la fois avec l'électrode de travail 5 et avec la contre-électrode 7, qui peut recevoir la solution introduite dans la cellule électrochimique d'oxydation (A) pour y subir une oxydation ou une oxydoréduction.

Tel que représenté sur la figure, une électrode de pseudo-référence 10 est disposée sur l'une 5a des faces de l'électrode de travail 5, précisément la face 5a placée en vis-à-vis de la contre-électrode 7.

La électrode de pseudo-référence 10 est disposée de préférence sur au moins une partie du pourtour de la face 5a de l'électrode de travail 5, avec une extension 11, au niveau de du connecteur 6 de l'électrode de travail 5, qui constitue un connecteur 11 à une source de potentiel électrique.

L'électrode de travail 5 et l'électrode de pseudo-référence 10 sont séparées par une couche isolante, déposée de préférence par sérigraphie sur la surface 5a de l'électrode de travail 5, pour éviter le contact électrique entre ces deux électrodes 10 et 5.

Ainsi, la cellule électrochimique d'oxydation (A) forme un compartiment dans lequel les produits d'oxydation d'une entité chimique tel qu'un xénobiotique peuvent être générés.

La production des espèces intermédiaires peut être obtenue par des balayages successifs des potentiels avec une vitesse pouvant aller de quelques mV/s à 10 V/s dans une fenêtre de potentiel variable et choisie en fonction des potentiels rédox du xénobiotique étudié et de ceux des espèces générées.

L'utilisation des balayages des potentiels combinée avec une disposition parallèle de l'électrode de travail 5 et de la contre-électrode 7 permet d'obtenir des processus faradiques (anodiques et cathodiques) de durée variable et alternés sur les deux surfaces d'électrodes en vis-à-vis.

Ceci permet d'obtenir un mélange de produits oxydés et réduits. Ce procédé est fort utile dans le cas de molécules pouvant subir une coupure électrochimique (N-déslakylation, S-déslakylation, O-déslakylation, etc.) suivie de formation d'espèces instables sous leurs formes oxydées telles que les quinones, quinone-imines ou quinone-methide.

Le fait d'alterner les processus anodiques et cathodiques par balayage des potentiels, permet de minimiser la réaction d'hydrolyse de certaines quinone-imines ou quinonemethides en benzoquinone.

La deuxième cellule électrochimique (B), ou cellule électrochimique de stabilisation (B), permet, lorsqu'elle est soumise à un potentiel électrochimique, la réduction d'une solution.

La structure de cette cellule électrochimique de stabilisation (B) est symétrique de celle de la cellule électrochimique d'oxydation (A), par rapport à l'élément central 2 du corps (C).

On retrouve donc dans cette cellule électrochimique de stabilisation (B) les même éléments que ceux décrits relativement à la cellule électrochimique d'oxydation (A), à savoir : l'électrode de travail 5' de forme sensiblement rectangulaire avec son connecteur 6' ; l'électrode de pseudo-référence 10' avec son connecteur 11', sur le pourtour et à la surface 5a' de l'électrode de travail 5' ; la contre-électrode 7' de forme sensiblement rectangulaire avec son connecteur 8', en vis-à-vis de l'électrode de travail 5' ; l'élément d'espacement 9' entre l'électrode de travail 5' et la contre-électrode 7', définissant un volume pouvant recevoir une solution introduite dans la cellule électrochimique de stabilisation (B) pour y subir une réduction ou une oxydoréduction.

De préférence, la contre-électrode 7' de la cellule électrochimique de stabilisation (B) est recouverte entièrement d'un film poreux sur sa surface en vis-à-vis de l'électrode de travail 5', ce qui permet de minimiser les processus faradiques anodiques dans cette cellule électrochimique de stabilisation (B).

Les électrodes de travail 5, 5' et contre-électrodes 7, 7' sont de préférence préparées par dépôts physiques ou chimiques en utilisant des réacteurs à plasma, ou encore par l'intermédiaire de procédés de sérigraphie.

Dans les deux cas, un film conducteur est déposé sur un support en céramique ou en inox. Les matériaux d'électrodes utilisés (de type film) sont de préférence les suivants : carbone, graphite, platine et or.

Les électrodes de pseudo-référence 10, 10', quant à elles, sont de préférence préparées à partir d'une encre composite d'argent (Ag) ou de palladium (Pd).

La disposition de l'ensemble des électrodes au sein de chaque cellule électrochimique (A) et (B), de façon sensiblement parallèle les unes aux autres, permet d'assurer un flux continu de la solution à électrolyser à l'intérieur de ces cellules.

La cellule électrochimique d'oxydation (A) et la cellule électrochimique de stabilisation (B) sont reliées en série l'une à l'autre.

Dans l'exemple représenté sur la figure, cette liaison en série est obtenue notamment par un jeu d'orifices dans la plaque supérieure 3 fermant le corps (C).

Ainsi, cette plaque supérieure 3, disposée du côté des contre-électrodes 7, 7' est pourvue d'un orifice d'entrée 12 permettant l'introduction d'une solution dans la cellule électrochimique d'oxydation (A), et d'un orifice de sortie 13 permettant la sortie d'une solution introduite dans la cellule électrochimique d'oxydation (A) après que cette solution a subi une oxydation dans cette cellule électrochimique d'oxydation (A).

Par ailleurs, la plaque supérieure 3, est pourvue d'un orifice d'entrée 12' permettant l'introduction d'une solution dans la cellule électrochimique de stabilisation (B), et d'un orifice de sortie 13' permettant la sortie d'une solution introduite dans la cellule électrochimique de stabilisation (B) après que cette solution a subi une réduction dans cette cellule électrochimique de stabilisation (B).

Un élément de connexion 14 relie l'orifice de sortie 13 de la cellule électrochimique d'oxydation (A) à l'orifice d'entrée 12' de la cellule électrochimique de stabilisation (B).

Pour faciliter le maintien de l'élément de connexion 14 en position, on peut utiliser des embouts 13a, 12a' tels que des vis percées qui viennent se viser par l'une de leurs extrémités dans les orifices respectifs 13, 12' et qui reçoivent par leur autre extrémité les extrémités respectives de l'élément de connexion 14.

Le principe peut être identique pour l'élément 15 permettant d'amener la solution dans la cellule électrochimique d'oxydation (A) via un embout 12a dans l'orifice 12, ainsi que pour l'élément 16 permettant l'évacuation de la solution de la cellule électrochimique de stabilisation (B) via un embout 13a' dans l'orifice 13'.

De préférence, un élément d'étanchéité 17 est disposé dans la cellule électrochimique d'oxydation (A), entre la plaque supérieure 3 et la contre-électrode 7. Cet élément d'étanchéité 17 est pourvu de deux trous traversants 17a et 17b respectivement en vis-à-vis de l'orifice d'entrée 12 et de l'orifice de sortie 13, ces trous traversants 17a et 17b débouchant par ailleurs respectivement dans des trous 7a et 7b de la contre-électrode 7.

De même, et également de préférence, un élément d'étanchéité 17' est disposé dans la cellule électrochimique de stabilisation (B), entre la plaque supérieure 3 et la contre-électrode 7'. Cet élément d'étanchéité 17' est pourvu de deux trous traversants 17a' et 17b' respectivement en vis-à-vis de l'orifice d'entrée 12' et de l'orifice de sortie 13', ces trous traversant 17a' et 17b' débouchant par ailleurs respectivement dans des trous 7a' et 7b' de la contre-électrode 7'

De préférence, des éléments de fixation 18, 18' viennent s'insérer, par exemple par vissage, dans la plaque inférieure 4, côté électrodes de travail 5, 5', de sorte à assurer la fixation de ces électrodes de travail 5 et 5' dans leur cellules électrochimiques respectives (A) et (B).

Ainsi, la cellule électrochimique de stabilisation (B), reliée en série à la cellule électrochimique d'oxydation (A), constitue un compartiment cathodique (B) permettant la réduction électrochimique des espèces oxydées générées dans la cellule électrochimique d'oxydation (A). Cette réduction électrochimique permet à son tour de stabiliser ces espèces oxydées sous leur forme réduite et ainsi de les récupérer en quantité suffisante.

Une application du dispositif et du procédé tels que décrits ci-dessus à la dégradation oxydative d'un β-bloquant tel que la molécule d'acébulotol, a donné les résultats présentés ci-après.

L'acébulotol introduit en flux continu dans la cellule électrochimique d'oxydation (A) subit une réaction de coupure anodique irréversible.

Le balayage successif des potentiels à une vitesse supérieure à 1 V/s dans une fenêtre de potentiels de -0.4 à 0.9 V vs Pd/H₂, permet la formation non seulement de l'espèce instable quinoneimine mais également celle de son état réduit 222 en quantité significative.

En effet, l'électrode de travail 5 et la contre-électrode 7 disposées parallèlement l'une à l'autre dans un flux continu de la solution à électrolyser, sont le siège de processus faradiques à la fois anodiques et cathodiques qui se déroulent dans la cellule électrochimique d'oxydation (A), tels que schématisés ci-dessous :

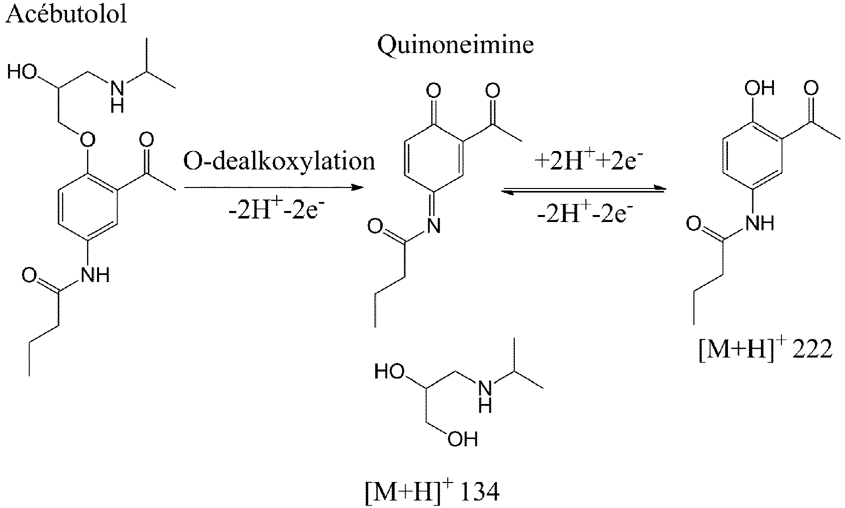

Ainsi, la stabilité de l'espèce quinoneimine instable est augmentée, et la durée des processus cathodiques se déroulant dans la cellule électrochimique de stabilisation (B) est réduite.

En effet, la cellule électrochimique de stabilisation (B) permet une réduction continue de l'espèce quinone imine au fur et à mesure de sa formation dans la cellule électrochimique d'oxydation (A).

A la fin de la synthèse, l'espèce intermédiaire est récupérée sous forme stable m/z 222, qui est une espèce plus facile à isoler en quantité suffisante, à l'échelle de la centaine de mg, avec un rendement qui peut être de l'ordre de 47%.

Il est rappelé que l'ensemble de la description ci-dessus est donné à titre d'exemple et n'est pas limitatif de l'invention.

En particulier, la forme des électrodes n'est pas limitative de l'invention, même si la forme sensiblement rectangulaire est préférée pour les électrodes de travail 5, 5' et contre-électrodes 7, 7'.

De même, la forme des logements 1, 1' recevant respectivement les cellules électrochimiques d'oxydation (A) et de stabilisation (B), et la forme de l'élément central 2 du corps (C) du dispositif, ne sont pas limitatives de l'invention

## Revendications

1. Dispositif de synthèse d'espèces intermédiaires d'une entité chimique par voie électrochimique, comprenant une cellule électrochimique d'oxydation (A) comportant au moins une première électrode de travail (5) et une première contre-électrode (7), et étant apte, lorsque lesdites premières électrode de travail (5) et contre-électrode (7) sont soumises à un potentiel électrique, à générer lesdites espèces intermédiaires par oxydation d'une solution introduite dans ladite cellule électrochimique d'oxydation (A) et comprenant ladite entité chimique, ledit dispositif comprenant également une cellule électrochimique de stabilisation (B) comportant au moins une deuxième électrode de travail (5') et une deuxième contre-électrode (7'), et étant apte, lorsque lesdites deuxièmes électrode de travail (5') et contre-électrode (7') sont soumises à un potentiel électrique, à réaliser une réduction d'une solution, cette dite cellule électrochimique de stabilisation (B) étant reliée en série à la cellule électrochimique d'oxydation (A), les deuxièmes électrode de travail (5') et contre-électrode (7') étant respectivement distinctes des premières électrode de travail (5) et contre électrode (7), en sorte de permettre la réduction continue dans la cellule électrochimique de stabilisation (B) des espèces intermédiaires générées dans la cellule électrochimique d'oxydation (A) par balayage, dans une fenêtre de potentiels donnée, de potentiels électriques appliqués aux premières électrode de travail (5) et contre-électrode (7), et **caractérisé en ce que** les premières électrode de travail (5) et contre-électrode (7) sont disposées de façon sensiblement parallèles l'une par rapport à l'autre, et maintenues espacées l'une de l'autre au moyen d'un premier élément d'espacement (9), tel qu'un joint de silicone, de façon à présenter chacune une surface en vis-à-vis de l'autre.

2. Dispositif selon la revendication 1, **caractérisé en ce que** l'épaisseur du premier élément d'espacement (9) est comprise entre 0,4 et 1,1 mm.

3. Dispositif selon l'une quelconque des revendications 1 et 2, **caractérisé en ce que** les électrodes de travail (5, 5') et/ou la ou les contre-électrodes (7, 7') sont de forme sensiblement rectangulaire.

4. Dispositif selon l'une quelconque des revendications 1 à 3, **caractérisé en ce que** la cellule électrochimique d'oxydation (A) et/ou la cellule électrochimique de stabilisation (B) comprennent une électrode de pseudo-référence (10, 10').

5. Dispositif selon la revendication 4, **caractérisé en ce que** la ou les électrodes de pseudo-référence (10, 10') sont disposées sur l'une des faces (5a, 5a') des électrodes de travail (5, 5') respectives, de préférence sur tout ou partie du pourtour de l'une des faces (5a, 5a') de ces électrodes de travail (5, 5'), et isolées électriquement de ces dernières par des couches isolantes respectives, lesdites couches isolantes étant par exemple déposées par sérigraphie.

6. Dispositif selon l'une quelconque des revendications 1 à 5, **caractérisé en ce qu'**il comprend un corps (C) comprenant un premier et un deuxième logements (1, 1') destinés à recevoir les cellules électrochimiques respectivement d'oxydation (A) et de stabilisation (B).

7. Dispositif selon la revendication 6, **caractérisé en ce que** les premier et deuxième logements (1, 1') sont formés par deux espaces (1, 1') disposés de part et d'autre d'un élément central (2) en forme de « H ».

8. Dispositif selon l'une quelconque des revendications 6 et 7, **caractérisé en ce que** le corps (C) est fermé par une plaque supérieure (3) du côté des contre-électrodes (7, 7'), et par une plaque inférieure (4) du côté des électrodes de travail (5, 5').

9. Dispositif selon la revendication 8, **caractérisé en ce que** la plaque supérieure (3) est pourvue d'une part d'un orifice d'entrée (12) dans, et d'un orifice de sortie (13) de, la cellule électrochimique d'oxydation (A), et d'autre part d'un orifice d'entrée (12') dans, et d'un orifice de sortie (13') de, la cellule électrochimique de stabilisation (B), ledit orifice de sortie (13) de la cellule électrochimique d'oxydation (A) étant relié audit orifice d'entrée (12') dans la cellule électrochimique de stabilisation (B).

10. Dispositif selon l'une quelconque des revendications 8 et 9, **caractérisé en ce que** la plaque inférieure (4) est pourvue de deux éléments de fixation (18, 18") aptes à permettre la fixation respectivement des première et deuxième électrodes de travail (5, 5').

11. Procédé de synthèse d'espèces intermédiaires d'une entité chimique par voie électrochimique comprenant une étape de génération des espèces intermédiaires par oxydation d'une solution contenant ladite entité chimique dans une cellule électrochimique d'oxydation (A), et une étape de stabilisation des espèces intermédiaires générées par réduction dans une cellule électrochimique de stabilisation (B) reliée en série à, la cellule électrochimique d'oxydation (A), où la génération des espèces intermédiaires dans la cellule électrochimique d'oxydation (A) comprend le balayage, dans une fenêtre de potentiels donnée, de potentiels électriques appliqués à une première électrode de travail (5) et une première contre-électrode (7) disposées de façon sensiblement parallèles l'une par rapport à l'autre, et maintenues espacées l'une de l'autre au moyen d'un premier élément d'espacement (9), tel qu'un joint de silicone, de façon à présenter chacune une surface en vis-à-vis de l'autre, et où la stabilisation, dans la cellule électrochimique de stabilisation (B), des espèce intermédiaires générées dans la cellule électrochimique d'oxydation (A), comprend la réduction continue de ces espèces intermédiaires par application d'un potentiel électrique à une deuxième électrode de travail (5') et une deuxième contre-électrode (7') respectivement distinctes des premières électrode de travail (5) et contre-électrode (7).

## Patentansprüche

1. Vorrichtung zur Synthese von Zwischenarten einer chemischen Einheit auf elektrochemischem Wege, über eine elektrochemische Oxidationszelle (A) verfügend, die mindestens eine erste Arbeitselektrode (5) und eine erste Gegenelektrode (7) hat und dazu geeignet ist, wenn die erste Arbeitselektrode (5) und die erste Gegenelektrode (7) einem elektrischen Potential ausgesetzt werden, die Zwischenarten durch Oxidation einer Lösung zu erzeugen, die in die elektrochemische Oxidationszelle (A) eingebracht ist und die chemische Einheit enthält; wobei die Vorrichtung auch über eine elektrochemische Stabilisierungszelle (B) verfügt, die mindestens eine zweite Arbeitselektrode (5') und eine zweite Gegenelektrode (7') hat und dazu geeignet ist, wenn die zweite Arbeitselektrode (5') und die zweite Gegenelektrode (7') einem elektrischen Potential ausgesetzt werden, eine Reduktion einer Lösung zu bewerkstelligen, wobei diese elektrochemische Stabilisierungszelle (B) in Reihe mit der elektrochemischen Oxidationszelle (A) geschaltet ist,
wobei sich die zweite Arbeitselektrode (5') und die zweite Gegenelektrode (7') jeweils von der ersten Arbeitselektrode (5) und der ersten Gegenelektrode (7) unterscheiden, um die kontinuierliche Reduktion von in der elektrochemischen Oxidationszelle (A) erzeugten Zwischenarten in der elektrochemischen Stabilisierungszelle (B) durch Abtasten, in einem bestimmten Fenster von Potentialen, von elektrischen Potentialen zuzulassen, die an die erste Arbeitselektrode (5) und die erste Gegenelektrode (7) angelegt werden, und
**dadurch gekennzeichnet, dass** die erste Arbeitselektrode (5) und die erste Gegenelektrode (7) im Wesentlichen parallel zueinander angeordnet sind und mittels eines ersten Beabstandungselements (9), wie etwa einer Silikondichtung, so beabstandet voneinander gehalten werden, dass sie jeweils eine einander zugewandte Fläche aufweisen.

2. Vorrichtung nach Anspruch 1, **dadurch gekennzeichnet, dass** die Dicke des ersten Beabstandungselements (9) zwischen 0,4 und 1,1 mm beträgt.

3. Vorrichtung nach einem der Ansprüche 1 und 2, **dadurch gekennzeichnet, dass** die Arbeitselektroden (5, 5') und/oder die Gegenelektrode/n (7, 7') von im Wesentlichen rechteckiger Form sind.

4. Vorrichtung nach einem der Ansprüche 1 bis 3, **dadurch gekennzeichnet, dass** die elektrochemische Oxidationszelle (A) und/oder die elektrochemische Stabilisierungszelle (B) eine Pseudo-Referenzelektrode (10, 10') aufweisen.

5. Vorrichtung nach Anspruch 4, **dadurch gekennzeichnet, dass** die Pseudo-Referenzelektrode/n (10, 10') an einer der Flächen (5a, 5a') der jeweiligen Arbeitselektroden (5, 5') vorzugsweise über den gesamten oder einen Teil des Umfangs einer der Flächen (5a, 5a') dieser Arbeitselektroden (5, 5') angeordnet und elektrisch durch jeweilige isolierende Schichten von diesen Letzteren isoliert sind, wobei die isolierenden Schichten zum Beispiel durch Siebdruck aufgetragen sind.

6. Vorrichtung nach einem der Ansprüche 1 bis 5, **dadurch gekennzeichnet, dass** sie einen Körper (C) mit einer ersten und zweiten Aufnahme (1, 1') aufweist, die dazu bestimmt sind, die elektrochemische Oxidationszelle (A) bzw. die elektrochemische Stabilisierungszelle (B) aufzunehmen.

7. Vorrichtung nach Anspruch 6, **dadurch gekennzeichnet, dass** die erste und zweite Aufnahme (1, 1') durch zwei Räume (1, 1') gebildet sind, die beidseits eines "H"-förmigen mittleren Elements (2) angeordnet sind.

8. Vorrichtung nach einem der Ansprüche 6 und 7, **dadurch gekennzeichnet, dass** der Körper (C) auf der Seite der Gegenelektroden (7, 7') mit einer oberen Platte (3) und auf der Seite der Arbeitselektroden (5, 5') mit einer unteren Platte (4) verschlossen ist.

9. Vorrichtung nach Anspruch 8, **dadurch gekennzeichnet, dass** die obere Platte (3) einerseits mit einer Eintrittsöffnung (12) in und einer Austrittsöffnung (13) aus der elektrochemischen Oxidationszelle (A) und andererseits mit einer Eintrittsöffnung (12') in und einer Austrittsöffnung (13') aus der elektrochemischen Stabilisierungszelle (B) versehen ist, wobei die Austrittsöffnung (3) der elektrochemischen Oxidationszelle (A) mit der Eintrittsöffnung (12') in die elektrochemische Stabilisierungszelle (B) verbunden ist.

10. Vorrichtung nach einem der Ansprüche 8 und 9, **dadurch gekennzeichnet, dass** die untere Platte (4) mit zwei Befestigungselementen (18, 18") versehen ist, die sich dazu eignen, die Befestigung der ersten bzw. zweiten Arbeitselektrode (5, 5') zuzulassen.

11. Verfahren zur Synthese von Zwischenarten einer chemischen Einheit auf elektrochemischem Wege, einen Schritt zum Erzeugen der Zwischenarten durch Oxidation einer die chemische Einheit enthaltenden Lösung in einer elektrochemischen Oxidationszelle (A), und einen Schritt zur Stabilisierung der durch Reduktion erzeugten Zwischenarten in einer elektrochemischen Stabilisierungszelle (B) umfassend, die mit der elektrochemischen Oxidationszelle (A) in Reihe geschaltet ist,
wobei das Erzeugen der Zwischenarten in der elektrochemischen Oxidationszelle (A) das Abtasten, in einem bestimmten Fenster von Potentialen, von elektrischen Potentialen umfasst, die an eine erste Arbeitselektrode (5) und eine erste Gegenelektrode (7) angelegt werden, die im Wesentlichen parallel zueinander angeordnet sind und mittels eines ersten Beabstandungselements (9), wie etwa einer Silikondichtung, so beabstandet voneinander gehalten werden, dass sie jeweils eine einander zugewandte Fläche aufweisen, und wobei die Stabilisierung, in der elektrochemischen Stabilisierungszelle (B), der in der elektrochemischen Oxidationszelle (A) erzeugten Zwischenarten die kontinuierliche Reduktion dieser Zwischenarten durch Anlegen eines elektrischen Potentials an eine zweite Arbeitselektrode (5') und eine zweite Gegenelektrode (7') umfasst, die sich jeweils von der ersten Arbeitselektrode (5) und der ersten Gegenelektrode (7) unterscheiden.

## Claims

1. A device for synthesizing intermediate species of a chemical entity electrochemically, comprising an electrochemical oxidation cell (A) including at least one first working electrode (5) and a first counter-electrode (7), and being capable, when said first working electrode (5) and counter-electrode (7) are subject to an electric potential, of generating said intermediate species by oxidation of a solution introduced into said electrochemical oxidation cell (A) and comprising said chemical entity, said device also comprising an electrochemical stabilization cell (B) including at least one second working electrode (5') and a second counter-electrode (7'), and being capable, when said second working electrode (5') and counter-electrode (7') are subject to an electric potential, of achieving reduction of a solution, this said electrochemical stabilization cell (B) being connected in series to the electrochemical oxidation cell (A), the second working electrode (5') and counter-electrode (7') being respectively distinct from the first working electrode (5) and counter-electrode (7), so as to allow continuous reduction in the electrochemical stabilization cell (B) of the intermediate species generated in the electrochemical oxidation cell (A) by scanning, in a given window of potentials, electric potentials applied to the first working electrode (5) and counter-electrode (7)
**characterized in that** the first working electrode (5) and counter-electrode (7) are positioned substantially parallel with respect to each other, and maintained spaced apart from each other by means of a first spacer element (9), such as a silicone gasket, so as to each have a surface facing each other.

2. The device according to claim 1, **characterized in that** the thickness of the first spacer element (9) is comprised between 0.4 and 1.1 mm.

3. The device according to any of claims 1 and 2, **characterized in that** the working electrodes (5, 5') and/or the counter-electrodes (7, 7') are of a substantially rectangular shape.

4. The device according to any of claims 1 to 3, **characterized in that** the electrochemical oxidation cell (A) and/or the electrochemical stabilization cell (B) comprise a pseudo-reference electrode (10, 10').

5. The device according to claim 4, **characterized in that** the pseudo-reference electrode(s) (10, 10') is(are) placed on one of the faces (5a, 5a') of the respective working electrodes (5, 5'), preferably on all or part of the perimeter of one of the faces (5a, 5a') of these working electrodes (5, 5'), and electrically insulated from the latter by respective insulating layers, said insulating layers for example being deposited by screen printing.

6. The device according to any of claims 1 to 5, **characterized in that** it comprises a body (C) comprising first and second housings (1, 1') intended to receive the electrochemical oxidation (A) and stabilization (B) cells, respectively.

7. The device according to claim 6, **characterized in that** the first and second housings (1, 1') are formed by two spaces (1, 1') positioned on either side of an "H-shaped" central element (2).

8. The device according to any of claims 6 and 7, **characterized in that** the body (C) is closed by an upper plate (3) on the side of the counter-electrodes (7, 7'), and by a lower plate (4) on the side of the working electrodes (5, 5').

9. The device according to claim 8, **characterized in that** the upper plate (3) is provided with an inlet orifice (12) in, and with an outlet orifice (13) of the electrochemical oxidation cell (A), on the other hand and an inlet orifice (12') in, and with an outlet orifice (13') of, the electrochemical stabilization cell (B), said outlet orifice (13) of the electrochemical oxidation cell (A) being connected to said inlet orifice (12') in the electrochemical stabilization cell (B).

10. The device according to any of claims 8 and 9, **characterized in that** the lower plate (4) is provided with two attachment elements (18, 18") capable of allowing attachment of the first and second working electrodes (5, 5'), respectively.

11. A method for synthesizing intermediate species of an chemical entity electrochemically comprising a step for generating intermediate species by oxidation of a solution containing said chemical entity in an electrochemical oxidation cell (A), and a step for stabilizing the intermediate species generated by reduction in an electrochemical stabilization cell (B) connected in series with the electrochemical oxidation cell (A),
wherein the generation of the intermediate species in the electrochemical oxidation cell (A) comprises the scanning, in a given window of potentials, of electric potentials applied to a first working electrode (5) and a first counter-electrode (7) placed substantially parallel with respect to each other, and maintained spaced apart from each other by means of a first spacer element (9), such as a silicone gasket, so as to each have a surface facing each other, and wherein the stabilization in the electrochemical stabilization cell (B), of the intermediate species generated in the electrochemical oxidation cell (A), comprises continuous reduction of these intermediate species by applying an electric potential to a second working electrode (5') and a second counter-electrode (7') respectively distinct from the first working electrode (5) and counter-electrode (7).
